# EUROPÄISCHE PATENTSCHRIFT

(11) **EP 3 912 803 B1**
(45) Veröffentlichungstag und Bekanntmachung des Hinweises auf die Patenterteilung: **06.09.2023**
(21) Anmeldenummer: 21170489.5
(22) Anmeldetag: 29.11.2016
(51) Int. Cl.: B29C 65/20, B29C 65/74, B29C 65/30, A61M 39/14, B29C 65/78

(54) **VORRICHTUNG ZUM SCHWEISSEN VON SCHLÄUCHEN**
DEVICE FOR WELDING HOSES
DISPOSITIF DE SOUDAGE DE TUYAUX

(30) Priorität: 30.11.2015 CH 17512015
(43) Veröffentlichungstag der Anmeldung: 24.11.2021
(62) Teilanmeldung aus: 16804752.0
(73) Patentinhaber: Reed Electronics AG, 6105 Schachen (CH)
(72) Erfinder: Bühler, Peter, 6103 Schwarzenberg (CH); Fluder, Willy, 6102 Malters (CH); Wyss, Markus, 6103 Schwarzenberg (CH)
(74) Vertreter: Rentsch Partner AG

(56) Entgegenhaltungen:
- EP-A1- 0 643 975

## Beschreibung

Die Erfindung betrifft ein Schweissmesser sowie eine Box umfassend eine Vielzahl von darin bevorrateten Schweissmessern

### STAND DER TECHNIK

Aus der US461 0670 A ist eine Vorrichtung und ein Verfahren zur sterilen Verbindung von zwei thermoplastischen Schläuchen bekannt. Bei diesem Verfahren werden die Enden zweier Schläuche miteinander verbunden, die jeweils mit einem Ende in einen Blutbeutel münden. Die beiden Schläuche werden hierzu parallel zu einander in zwei von einander beabstandete Blöcke eingelegt, mit einem beheizten Messer durchtrennt und zu einem durchgängigen Schlauch gefügt. Ziel ist es, eine sterile Verbindung zwischen zwei Schläuchen zu schaffen.

Die EP0208004 A1 offenbart ein Verfahren und eine Vorrichtung zum sterilen Docken von Kunststoffschlauchabschnitten. Vergleichbar mit der US4610670 A werden auch hier zwei Schlauchenden zu einem durchgängigen Schlauch gefügt mit dem Unterschied, dass zwei bereits geschnittene Schlauchenden in Halteblöcke eingespannt, durch eine Heizeinrichtung erwärmt und durch eine horizontale Verschiebung der Halteblöcke zueinander gefügt und miteinander verschweisst werden.

Die US 2012/0269679 A1 offenbart ein System mit welchem eine Vielzahl von Schläuchen, welche an ihrem einen Ende je mit einem Blutbeutel verbunden sind mit einer Vielzahl von Schläuchen, welche in einem sogenannten "Pooling-Container" münden, verschweisst werden. Im offenbarten Verfahren werden einzelne beheizte Klingen eingesetzt, um die Schläuche zu trennen und in einem weiteren Schritt miteinander zu verschweissen.

Die EP 0 643 975 A1 offenbart ein Schweissmesser für eine Vorrichtung zum Verschweissen von thermoplastischen Schläuchen, wobei das Schweissmesser eine rechteckige Form hat, an einer der beiden längeren Seiten des Rechtecks eine Klinge aufweist, elektrisch leitend ist, und wobei die Schmelzfläche des Schweissmessers zwei Ausnehmungen aufweist.

### DARSTELLUNG DER ERFINDUNG

Bei Verarbeitung von Blutkonserven kommen hoch automatisierte Prozessabläufe zum Einsatz. An die hierfür eingesetzte Maschine werden daher hohe Anforderungen bezüglich Effizienz und Sterilität gestellt. Ein wesentlicher Schritt ist das Verbinden mehrerer Blutbeutel miteinander, in dem die einzelnen Schläuche, welche von den einzelnen Beuteln wegführen, miteinander verschweisst werden. In Blutbanken werden zur Verbindung der einzelnen Blutkonserven pro Tag über 2000 Schweissungen an hunderten von Blutbeuteln vorgenommen, um beispielsweise aus Spenderblut Thrombozytenkonzentrat herzustellen. Es ist daher notwendig, möglichst viele Schweissungen innerhalb möglichst kurzer Zeit und von hoher und gleichbleibender Qualität durchführen zu können. Es soll eine sterile Verbindung hergestellt werden.

Eine Aufgabe ist es, ein beheizbares Schweissmesser zur Verfügung zu stellen, wobei eine Vielzahl von thermoplastischen Schläuchen geschnitten und deren Enden mit gleichbleibender Qualität verschweisst werden sollen. Die Aufgaben werden durch die Merkmale des Anspruchs 1 gelöst.

Bevorzugte Ausführungsarten der Erfindung sind in den abhängigen Ansprüchen angegeben.

Eine Vorrichtung zum Verschweissen von thermoplastischen Schläuchen einer ersten und zweiten Schlauchgruppe umfasst zwei Schlauchhalter mit je einer ersten und einer zweiten Schlauchklemme, sowie ein erfindungsgemässes Schweissmesser. Die Schläuche der ersten Schlauchgruppe verlaufen gerade durchlaufend zwischen den beiden Schlauchhaltern, werden in die Durchgangsöffnungen der ersten beiden Schlauchklemmen eingelegt und darin gequetscht.

Die Schläuche der zweiten Schlauchgruppe verlaufen ebenfalls gerade durchlaufend zwischen den beiden Schlauchhaltern, werden in Durchgangsöffnungen der beiden zweiten Schlauchklemmen eingelegt und darin gequetscht.

Das erfindungsgemässe Schweissmesser wird zwischen dem ersten und dem zweiten Schlauchhalter bewegt und ist dazu eingerichtet, die gequetschten Schläuche der ersten und der zweiten Schlauchgruppe in einer Bewegung zu durchtrennen, um auf diese Weise Schnitt-Enden und Rest-Enden zu erzeugen.

Einer der beiden Schlauchhalter kann relativ zum anderen Schlauchhalter verschoben werden, um dadurch die Schnitt-Enden der Schläuche aufeinander auszurichten.

Die beiden Schlauchhalter können ferner in einer horizontalen Richtung zu einander oder weg von einander verschoben werden, um dadurch je ein Schnitt-Ende eines Schlauchs der ersten Schlauchgruppe mit je einem Schnitt-Ende eines Schlauchs der zweiten Schlauchgruppe zu je einem durchgängigen Schlauch gleichzeitig zu verschweissen.

Ein Vorteil der Vorrichtung ist, dass auf diese Weise mit einer geringen Anzahl an Arbeitsschritten eine Vielzahl von thermoplastischen Schläuchen steril zu durchgängigen Schläuchen verschweisst werden können.

Die Durchgangsöffnungen der beiden ersten Schlauchklemmen auf einer ersten Ebene und die Durchgangsöffnungen der beiden zweiten Schlauchklemmen auf einer zweiten Ebene angeordnet, wobei die erste Ebene parallel und beabstandet zur zweiten Ebene angeordnet ist.

Die beiden ersten Schlauchklemmen und die beiden zweiten Schlauchklemmen weisen Quetschzonen auf, wobei diese Quetschzonen entlang der Durchgangsöffnungen verlaufen und dazu eingerichtet sind, die eingelegten Schläuche in einem schrägen Winkel zur ersten bzw. zur zweiten Ebene zu quetschen.

In einer bevorzugten Ausführungsform ist das Schweissmesser in einem Schweissmesserhalter einklemmbar und eine Antriebseinheit ist eingerichtet, den Schweissmesserhalter zum Durchtrennen der Schläuche in einer vertikalen Richtung zu bewegen. Bei dieser Bewegung in vertikaler Richtung wird einer der beiden Schlauchhalter relativ zum anderen Schlauchhalter zum Ausrichten der Schnitt-Enden der Schläuche vertikal verschoben. Des Weiteren werden durch die Bewegung des Schweissmesserhalters in vertikale Richtung die beiden Schlauchhalter in der horizontalen Richtung aufeinander zu oder voneinander weg oder beide Schlauchhalter gleichzeitig in eine Richtung bewegt

Die Vorrichtung weist eine in der vertikalen Richtung bewegbare Plattform auf und der Schweissmesserhalter ist auf dieser Plattform angeordnet.

In einer bevorzugten Ausführungsform weist der Schweissmesserhalter zwei sich gegenüberliegende Klemmteile auf, zwischen welche das Schweissmesser einklemmbar ist. Vorzugsweise ist eines der beiden Klemmteile feststehend und das andere relativ beweglich zum feststehenden Klemmteil angeordnet.

Ferner ist an einem der beiden Klemmteile ein Paar von ersten Kulissenträgern vorgesehen. In einer Ausführungsform ist das Klemmteil und das Paar von ersten Kulissenträgern als ein Stück gefertigt. Alternativ ist das Paar von ersten Kulissenträgern an einem der beiden Klemmteile als separate Teile montiert.

Das Paar von Kulissenträgern weist vorzugsweise insgesamt vier erste Kulissen auf. Diese Kulissen sind untereinander identisch und spiegelsymmetrisch zu einer Ebene des Schweissmessers angeordnet, wenn das Schweissmesser zwischen die beiden Klemmteile eingeklemmt ist.

Ferner sind Abtasträderpaare vorgesehen, die bei der Bewegung des Schweissmesserhalters in vertikaler Richtung die Kulissen abtasten und dabei die Schlauchhalter in der horizontalen Richtung zueinander oder weg voneinander oder beide Schlauchhalter gleichzeitig in eine Richtung bewegen.

Ferner sind ein zweiter Kulissenträger und ein weiteres Abtastrad vorgesehen. Das weitere Abtastrad ist dazu eingerichtet, bei der Bewegung des Schweissmesser-halters in vertikaler Richtung den zweiten Kulissenträger abzutasten. Dabei wird das eine Klemmteil relativ zum zweiten feststehenden Klemmteil bewegt und auf diese Weise der Abstand zwischen den beiden Klemmteilen variiert Beim Abtasten des zweiten Kulissenträgers durch das Abtastrad wird der Abstand zwischen den beiden Klemmteilen vorzugsweise in drei unterschiedlichen Positionen variiert. In einer Aufnahmeposition ist das Abtastrad in Kontakt mit dem zweiten Kulissenträger und es resultiert ein Abstand zwischen den beiden Klemmteilen, welcher gerade für das Einschieben des Schweissmessers benötigt wird In einer Kontaktierungsposition ist der Abstand zwischen den beiden Klemmteilen so gewählt, dass das Schweissmesser eingeklemmt und gleichzeitig elektrisch kontaktiert ist. In dieser Position ist das Abtastrad nicht in Kontakt mit dem zweiten Kulissenträger.

In einer Freigabeposition ist das Abtastrad in Kontakt mit dem zweiten Kulissenträger, und der Abstand zwischen den beiden Klemmteilen ist so gewählt, dass das Schweissmesser ausgeworfen werden kann.

Es sind an mindestens einem der beiden Klemmteile Druckstücke angeordnet, welche das Schweissmesser in der Kontaktierungsposition kontaktieren. In einer Ausführungsform sind diese Druckstücke an dem mindestens einen Klemmteil federnd gelagert. Durch die federnde Lagerung kann der Druck welcher bei der Kontaktierung auf das Schweissmesser ausgeübt wird, variabel eingestellt werden. Es hat sich herausgestellt, dass eine möglichst regelmässige Druckverteilung auf das Schweissmesser eine regelmässige Temperaturverteilung über die Fläche des Schweissmessers bewirkt. Eine möglichst gleichmässige Verteilung ist wünschenswert, da dies die Qualität der Verschweissung verbessert. Es ist dann sichergestellt, dass auf die Schnitt-Enden bzw. die Rest-Enden der thermoplastischen Schläuche eine vergleichbare Wärmeenergie, unabhängig von deren Lage im Schlauchhalter, aufgebracht wird.

Die Vorrichtung weist eine Transformatoreinheit mit einer Primärwicklung und einer Sekundärwicklung auf, wobei die Sekundärwicklung durch einen einzigen Leiter gebildet wird. Dieser Leiter ist mit einem der beiden Klemmteile, vorzugsweise mit dem feststehenden Klemmteil, verbunden. Das Schweissmesser schliesst in der Kontaktierungsposition den sekundären Stromkreis und bildet darin das Haupt-Widerstandelement, in dem die Verlustleistung überwiegend anfällt. Das Schweissmesser wird so auf die gewünschte Temperatur aufgeheizt. Das Schweissmesser wird mit einem hohen Wechselstrom auf eine Temperatur von rund 300 °C aufgeheizt. Mit der erfindungsgemässen Anordnung konnte eine konstante Heizleistung am Schweissmesser erzielt werden. Durch den erfindungsgemässen Aufbau kann die Elektrik gut in das Gehäuse der Vorrichtung integriert werden. Erfindungsgemäss weist die erste und die zweite Schlauchgruppe je die gleiche Anzahl an einzelnen Schläuchen, vorzugsweise mehr als zwei, insbesondere sechs Schläuche pro Schlauchgruppe, auf.

Das Verfahren zum Verschweissen einer Vielzahl von Schläuchen einer ersten und einer zweiten Schlauchgruppe mit der Vorrichtung umfasst mehrere Schritte.

Zu Beginn werden die Schläuche der ersten und der zweiten Schlauchgruppe parallel und übereinander in sich gegenüberliegende erste und zweite Schlauchhalter gerade durchlaufend eingelegt und gequetscht. Ein beheizbares Schweissmesser wird in einen Schweissmesserhalter eingelegt und auf eine gewünschte Temperatur erhitzt.

Das beheizte Schweissmesser wird zwischen dem ersten und dem zweiten Schlauchhalter bewegt, wobei die gequetschten Schläuche gleichzeitig in Schnitt-Enden und Rest-Enden durchtrennt werden.

Die Schnitt-Enden der Schläuche werden durch das beheizte Schweissmesser aufgeschmolzen.

Die Schnitt-Enden der Schläuche der ersten Schlauchgruppe werden auf die Schnitt-Enden der Schläuche der zweiten Schlauchgruppe ausgerichtet, in dem einer der beiden Schlauchhalter relativ zum anderen Schlauchhalter verschoben wird, sodass die Schnitt-Enden der Schläuche der ersten Schlauchgruppe symmetrisch gegenüberliegend zu den Schnitt-Enden der zweiten Schlauchgruppe angeordnet sind und das Schweissmesser herausgezogen werden kann.

Die beiden Schlauchhalter werden aufeinander zu verschoben, um dadurch je ein Schnitt-Ende der Schläuche der ersten Schlauchgruppe mit je einem Schnitt-Ende der Schläuche der zweiten Schlauchgruppe zu je einem durchgängigen Schlauch gleichzeitig zu verschweissen.

Zum Schluss wird das Schweissmesser aus dem Schweissmesserhalter ausgeworfen.

Beim Einlegen des Schweissmessers ist der horizontale Abstand zwischen dem ersten und dem zweiten Schlauchhalter d1. Beim Durchtrennen der gequetschten Schläuche wird der Abstand auf d4 vergrösserst. Beim Aufschmelzen der Schnitt-Enden beträgt der Abstand d2, wobei gilt d1<d2<d4. Beim Verschieben einer der beiden Schlauchhalter wird der Abstand wieder auf d4 vergrössert. Beim Verschweissen ist der Abstand d3, wobei gilt d3<d4 und beim Auswerfen des Schweissmessers beträgt der Abstand zwischen den beiden Schlauchhaltern d5, wobei d5 den kleinsten aller fünf Abstände darstellt.

Der Abstand d4 ist in seiner Grösse so gewählt, dass an den thermoplastischen Schläuchen eine Zugspannung anliegt. Ein Durchtrennen wird dadurch erleichtert. Beim Aufschmelzen der Schnitt-Enden wird der Abstand auf d2 verringert. Auf diese Weise berühren die Schnitt-Enden und die Rest-Enden das beheizte Schweissmesser und werden an Ihren Querschnitten entsprechend aufgeschmolzen. Beim Verschieben einer der beiden Schlauchhalter wird der Abstand wieder auf d4 vergrössert. Durch die Vergrösserung des Abstandes auf d4 ist sichergestellt, dass beim Herausziehen des Schweissmessers ausreichend Schmelzmaterial auf den Schlauchquerschnitten verbleibt, welches dann für die nachfolgende Verschweissung zur Verfügung steht. Danach werden je ein Schnitt-Ende eines Schlauchs der ersten Schlauchgruppe mit je einem Schnitt-Ende eines Schlauchs der zweiten Schlauchgruppe zu je einem durchgängigen Schlauch gleichzeitig verschweisst, in dem die Enden durch eine Verringerung des Abstandes zwischen den Schlauchhaltern auf d3 gefügt werden. Zum Schluss wird das Schweissmesser ausgeworfen und die verschweissten Schläuche entnommen. Die Schlauchhalter befinden sich im Abstand d5 zueinander, der im Regelfall so gering ist, dass sie einander berühren.

Das erfindungsgemässe Schweissmesser, welches bevorzugt in der als solchen nicht beanspruchten Vorrichtung bzw. beim als solchen nicht beanspruchten Verfahren zum Einsatz kommt, weist in seiner Schnittfläche eine Vielzahl von Ausnehmungen auf. Die Schnittfläche dient dazu die Schnitt-Enden bzw. die Rest-Enden der Schläuche aufzuschmelzen, weshalb im Folgenden von einer Schmelzfläche die Rede ist.

Es konnte festgestellt werden, dass die Ausnehmungen und insbesondere deren Anordnung und/oder Anzahl einen Einfluss auf die vertikale sowie horizontale Temperaturverteilung bezogen auf die Schweissmesserfläche hat. Eine regelmässige Verteilung ist vorteilhaft, da auf diese Weise ein möglichst gleichmässiges Aufschmelzen der Schnitt-Enden der thermoplastischen Schläuche sichergestellt ist und ein gutes Schweissergebnis sichergestellt wird.

In einer bevorzugten Ausführungsform weist die Schmelzfläche eine rechteckige Form auf und entlang der beiden kurzen Seiten des Rechtecks sind Randzonen vorgesehen, welche dazu eingerichtet sind, eine Sekundärseite einer Transformatoreinheit zu kontaktieren. Vorzugsweise erfolgt diese Kontaktierung über die Druckstücke, welche an einem der beiden Klemmteile angeordnet sind.

Die Ausnehmungen in der Schmelzfläche sind entlang der Randzonen der Schmelzfläche angeordnet.

Das erfindungsgemässe Schweissmesser weist an einer der beiden längeren Seiten des Rechtecks eine Klinge auf, wobei die Klinge bezogen auf den Querschnitt des Rechtecks eine symmetrische Form aufweist. Diese Form ist vorteilhaft, da das Schweissmesser beim Durchtrennen der Schläuche dadurch einen sehr geraden Schnitt erzeugt.

In einer Ausführungsform wird das Schweissmesser durch hälftige Längsfaltung eines Blättchens von doppelter Breite hergestellt. Als Klinge, welche die Schläuche schneidet, dient die Biegekante entlang welcher die beiden Hälften des Blättchens zusammenhängen. Auf diese Weise resultiert eine symmetrische abgerundete Klinge

Alternativ zur Faltung wird das Schweissmesser durch Stanzen und dessen Klinge durch Prägung hergestellt. Die Prägung wird entlang einer Längsseite des Blättchens vorgenommen, in dem die gewünschte Klingenform entlang der Längsseite eingepresst wird. Durch diese Gestaltung wird in rationeller Weise eine nicht zu scharfe, sondern abgerundete, vor allem jedoch gleichmässige und zu beiden Seiten hin symmetrische Schneidkante geschaffen.

Die erfindungsgemässen Schweissmesser werden in einer Box bevorratet, die vorzugsweise als Ganzes austauschbar ist.

### KURZE ERLÄUTERUNG ZU DEN FIGUREN

Die Erfindung soll nachfolgend anhand eines Ausführungsbeispiels im Zusammenhang mit der Zeichnung näher erläutert werden. Es zeigen:
- Fig. 1: zeigt eine perspektivische Ansicht der als solche nicht beanspruchten Vorrichtung in einer Ansicht von schräg oben mit Tisch und Tischbeinen,
- Fig. 1A: zeigt eine perspektivische Ansicht von oben auf die Vorrichtung mit geschlossenen Schlauchhaltern und eingelegten Schläuchen,
- Fig. 2: zeigt eine Ansicht auf die Vorrichtung von unten,
- Fig. 3: zeigt einen Teil der Vorrichtung in einer perspektivischen Ansicht mit geöffneten Schlauchhaltern,
- Fig. 4: zeigt eine Schnittdarstellung der Vorrichtung, wobei sich der Schweissmesserhalter in einer Position befindet, in welcher das Schweissmesser aufgenommen werden kann **("Bladefeed Position"),**
- Fig. 4A: zeigt die Vorrichtung in "Bladepickup Position", wobei einige Teile entfernt sind, so dass die Sicht auf die Schweissmesserzuführung und einen der Schweissmesserhalter frei ist,
- Fig. 4B: zeigt die Teildarstellung aus Fig. 4A im Zeitpunkt, in welchem das Schweissmesser in die Schweissmesserhalterung eingeführt ist,
- Fig. 5: zeigt eine Schnittdarstellung der Vorrichtung in einer Position, in welcher das Schweissmesser in die Schweissmesserhalterung eingelegt und elektrisch kontaktiert ist **("Precut Position"),**
- Fig. 6: zeigt eine Schnittdarstellung der Vorrichtung in einer Position, in welcher der erste und der zweite Schlauchhalter derart beabstandet voneinander angeordnet sind, dass die eingelegten Schläuche gedehnt werden **("Stretch Position")**,
- Fig. 7: zeigt eine Schnittdarstellung der als solcher nicht beanspruchten Vorrichtung in einer Position, in welcher die Schläuche durch das Schweissmesser durchtrennt werden und der erste und zweite Schlauchhalter in vertikaler Richtung gegen einander verschoben und die Schläuche aufeinander ausgerichtet sind **("Cut Position"),**
- Fig. 8: zeigt eine weitere Schnittdarstellung der Ausführungsform mit eingelegten Schläuchen, wobei der erste und der zweite Schlauchhalter in vertikaler Richtung gegeneinander verschoben und die zu verschweissenden Schlauchenden aufeinander ausgerichtet und gefügt sind **("Press Position")** und das Schweissmesser sich nicht mehr zwischen den Schlauchhaltern befindet,
- Fig. 9: zeigt eine Schnittdarstellung der Vorrichtung in einer Position, in welcher das Schweissmesser ausgeworfen wird **("Eject Position"),**
- Fig. 10A: zeigt eine bevorzugte Ausführungsform eines Schweissmessers,
- Fig. 11: zeigt die vertikale und horizontale Temperaturverteilung am Schweissmesser entlang einer ersten, zweiten und dritten Linie.

In den verschiedenen Figuren sind dieselben Teile mit denselben Bezugszeichen versehen. Von mehrfach vorhandenen, gleichartigen Teilen ist jeweils nur eines mit einem Bezugszeichen versehen.

### WEGE ZUR AUSFÜHRUNG DER ERFINDUNG

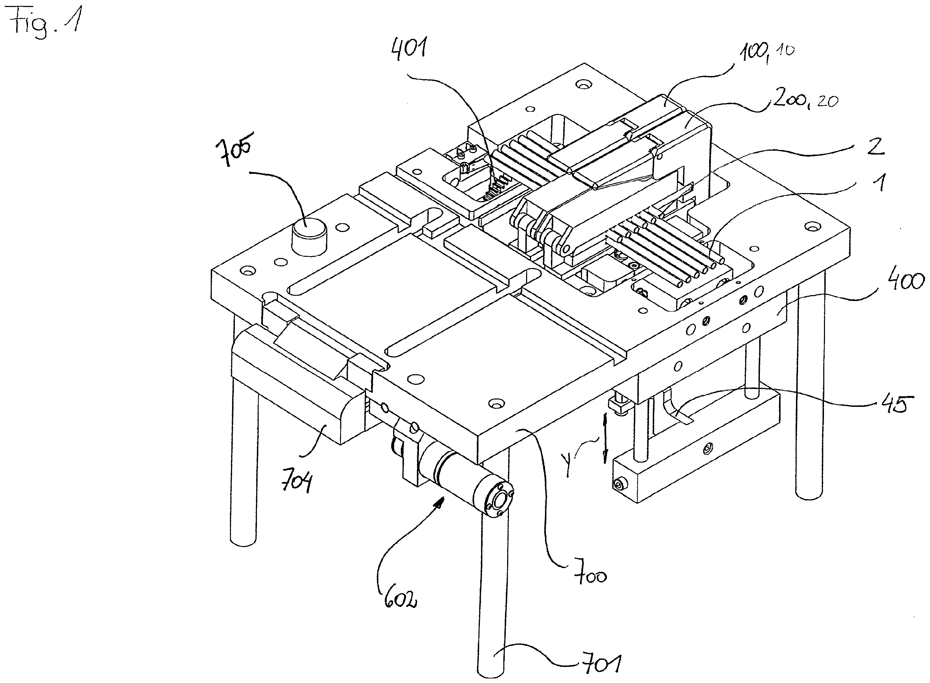

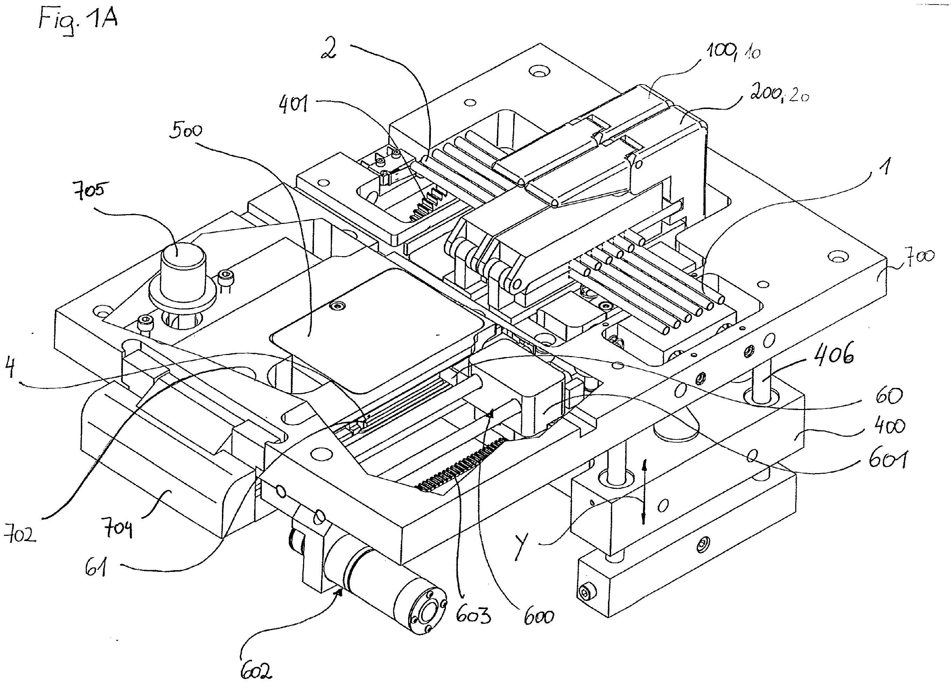

**Figur 1** zeigt eine perspektivische Ansicht von oben auf die als solche nicht beanspruchte Vorrichtung des Ausführungsbeispiels, die auf bzw. an einem Tisch 700 aufgebaut ist. Der Tisch steht auf vier Beinen 701. Die Vorrichtung weist zwei Schlauchhalter 100, 200 auf, in welche Schläuche eingelegt sind, wobei die Schläuche einer ersten Schlauchgruppe 1 auf einer ersten Ebene und die Schläuche einer zweiten Schlauchgruppe 2 auf einer zweiten Ebene darüber verlaufen. Die beiden Schlauchhalter 100, 200 weisen zusätzlich Spannhebel 10, 20 auf.

In Figur 1A ist durch einen zu diesem Zweck erzeugten Ausschnitt 702 des Tisches 700 eine Box 500 sichtbar, in welcher Schweissmesser bevorratet sind, und eine Schweissmesserzuführung 600 zu einem Schweissmesserhalter 40. Letzterer ist unterhalb der Schlauchhalter 100, 200 angeordnet, so dass er in beiden Figuren 1 und 1A nicht sichtbar ist. Die Schweissmesserzuführung 600 umfasst einen Transportschlitten 601 für einen Schieber 60 und einen Antrieb 602, bei dem es sich beispielsweise um einen "DC Planetary Gear Brush Motor" handelt. Der Schieber 60 ist mit dem Antrieb 602 über einen Zahnriemen 603 verbunden. Die Box 500 ist derart ausgestaltet, dass die einzelnen Schweissmesser 4 in Längsrichtung aufgestellt in der Box 500 einsortiert sind (in Figur 1 nicht sichtbar). Die Box 500 ist an jener Seite, welche zur Schweissmesserzuführung 600 hin orientiert ist, offen. Ein Federelement, angeordnet in der Box (in Figur 1 nicht sichtbar) drückt das Schweissmesser 4, welches für den nächsten Schweissvorgang vorgesehen ist, in die Führungsschienen 61 der Schweissmesserzuführung 600.

Unter dem Tisch 700 angeordnet ist eine Plattform 400, auf welcher der Schweissmesserhalter 40 (in Figur 1 nicht sichtbar) montiert ist. Die Plattform 400 und Schweissmesserhalter 40 kann mit Hilfe einer weiteren Antriebseinheit 401 auf den Tisch 700 zu bzw. von diesem weg bewegt werden (Pfeil Y), d. h. wenn der Tisch horizontal ausgerichtet ist, in vertikaler Richtung auf und ab.

In Figur 1 sowie in den weiteren Figuren ist auf die Darstellung eines üblicherweise zusätzlich vorhandenen Gehäuses verzichtet. Aus dem Gehäuse ragen bevorzugt die Schlauchhalter 100, 200 nach oben heraus, so dass das Gehäuse zum Einlegen oder zum Herausnehmen der Schläuche nicht geöffnet werden muss. Ausserdem sollte die Box 500 mit dem Schneidmesservorrat für einen Austausch einfach zugänglich sein, was mit Hilfe einer Schublade 704 möglich gemacht wird. Bedienelemente wie ein Startknopf 705 können ebenfalls an dem Gehäuse vorgesehen sein (siehe beispielsweise Figur 1, 1A).

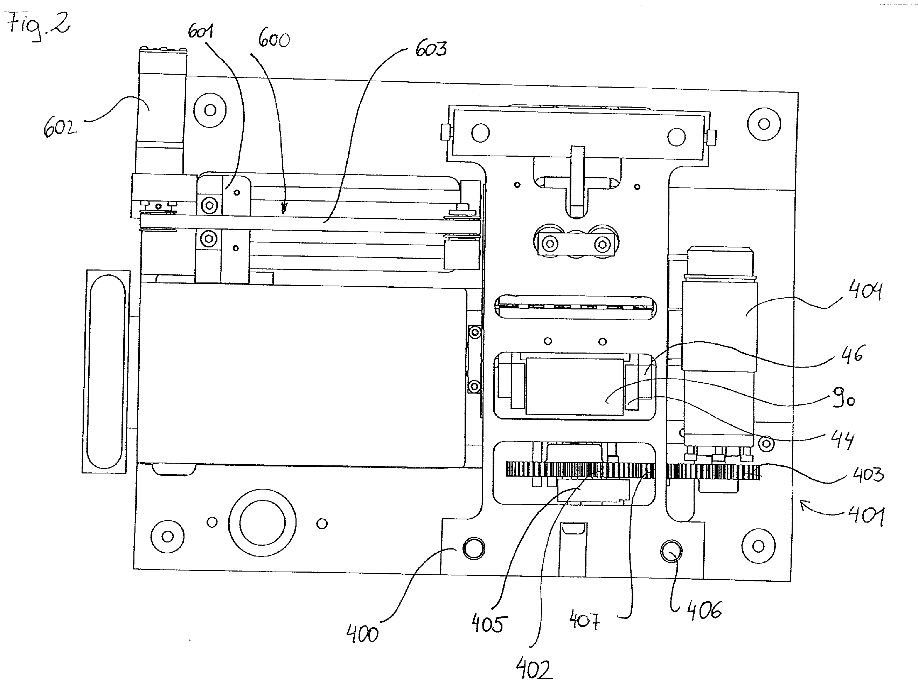

**Figur 2** zeigt die als solche nicht beanspruchte Vorrichtung in einer Ansicht von unten, wobei die Plattform 400, auf die der Schweissmesserhalter 40 aufgesetzt ist, und mehr von der Antriebseinheit 401 zu sehen ist, welche die Plattform 400 in einer vertikalen Richtung bewegt. Sie umfasst drei Zahnräder 402 403 und 407, wobei das Zahnrad 402 über ein Pleuel 405 mit der Plattform 400 verbunden ist. Das Zahnrad 407 befindet sich zwischen dem Zahnrad 402 und 403 (in Figur 2 sichtbar). Das Pleuel 405 setzt die kreisförmige Bewegung des Zahnrads 402 in eine lineare Auf- und Ab Bewegung der Plattform 400 entlang stangenförmiger Führungen 406 um. Das Zahnrad 403 wird durch einen Motor 404 angetrieben, bei dem es sich beispielsweise wieder um einen "DC Planetary Gear Brush Motor" handelt. In Fig. 2 sichtbar ist auch eine Transformatoreinheit 90 zur Heizung des Schweissmessers 4, die nachstehend noch näher beschrieben wird.

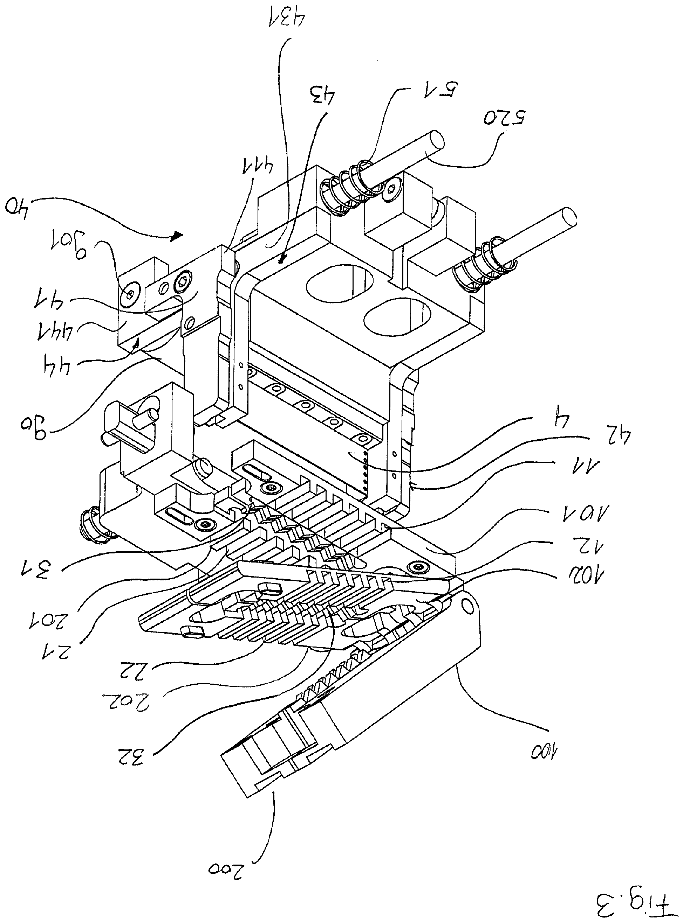

**Figur 3** zeigt einen Teil der Vorrichtung in einer perspektivischen Ansicht mit geöffneten Schlauchhaltern 100, 200, ohne eingelegte Schläuche. Der erste und der zweite Schlauchhalter 100, 200 umfassen je eine erste 101, 201 und eine zweite Schlauchklemme 102, 202. Die erste Schlauchklemme 101 des ersten Schlauchhalters 100 und die erste Schlauchklemme 201 des zweiten Schlauchhalters 200 weisen je ein Unterteil und ein Oberteil auf. Die zweite Schlauchklemme 102 des ersten Schlauchhalters 100 und die zweite Schlauchklemme 202 des zweiten Schlauchhalters 200 weisen ebenfalls je ein Unterteil und ein Oberteil auf. Das Oberteil der ersten Schlauchklemme 101 des ersten Schlauchhalters 100 und das Oberteil der ersten Schlauchklemme 201 des zweiten Schlauchhalters 200 bilden gleichzeitig das Unterteil der zweiten Schlauchklemme 102, 202 des ersten bzw. zweiten Schlauchhalters 100, 200. Jede Schlauchklemme weist Durchgangsöffnungen 11, 12, 21 und 22 auf, in welche die Schläuche einer ersten und einer zweiten Schlauchgruppe eingelegt werden. Die Durchgangsöffnungen 11, 21 der beiden ersten Schlauchklemmen 101, 201 sind auf einer ersten Ebene und die Durchgangsöffnungen 12, 22 auf einer zweiten Ebene angeordnet. Die erste Ebene ist parallel und vertikal beabstandet zur zweiten Ebene. Die beiden ersten Schlauchklemmen 101, 201 sowie die beiden zweiten Schlauchklemmen 102, 202 weisen erste und zweite Quetschzonen 31, 32 auf, welche dazu eingerichtet sind, die eingelegten Schläuche vor dem Durchtrennen zu quetschen. Die Quetschzonen 31, 32 sind sägezahnförmig ausgebildet, so dass die Schläuche unter einem schrägen Winkel, insbesondere von 25°- 30° gequetscht werden. Durch die Quetschung werden die Schläuche komplett verschlossen, so dass insbesondere in den Schläuchen enthaltene Flüssigkeit beispielsweise von Blutkonserven die Quetschzonen nicht mehr passieren kann.

In der Figur 3 sichtbar ist weiter der Schweissmesserhalter 40 mit einem ersten Klemmteil 43, welches gegenüberliegend zu einem zweiten Klemmteil 44 angeordnet ist und relativ zu diesem zweiten, feststehenden Klemmteil entlang von stangenförmigen Führungen 520 beweglich ist. Die beiden Klemmteile weisen jeweils ein Paar von abgewinkelten Klemmarmen 431, 441 auf, wobei das Schweissmesser 4 zwischen den nach oben aufragenden Abschnitten dieser Klemmarme gehalten wird. Zum Anpressen des ersten Klemmteils 43 gegen das zweite Klemmteil 44 kommen erste Federn 51 zum Einsatz.

Am Klemmteil 44 befestigt ist ein Paar von Kulissenträgern 41, an deren aufragenden Abschnitten beidseitig und damit insgesamt vier erste Kulissen 411 ausgeformt sind. Alternativ ist das Klemmteil 44 und das Paar von Kulissenträgern 41 als ein Teil ausgebildet. Die Kulissen 411 sind untereinander identisch und spiegelsymmetrisch zur Ebene des Schweissmessers 4 angeordnet, wenn dieses zwischen den Klemmteilen 43, 44 eingeklemmt ist. Weiter ist in dem in Figur 3 hinteren Kulissenträger 41 ein Schlitz 42 für das Einschieben des Schweissmessers 4 zwischen die beiden Klemmteile 43, 44 vorhanden (in Figur 3 nicht sichtbar).

In der Figur 3 sind auch weitere Teile einer Heizung für das Schweissmesser 4 sichtbar, welche die bereits erwähnte Transformatoreinheit 90 umfasst. Um einen hohlzylindrischen Ferritkern dieser Einheit ist über dessen Umfang verteilt eine Primärwicklung durch mehrere Windungen Kupferdraht, beispielsweise 5-15 Windungen, gewickelt, wobei sich die einzelnen Windungen im Wesentlichen in Axialrichtung des Ferritkerns sowie durch diesen hindurch erstrecken. Die Sekundärwicklung wird demgegenüber nur durch einen einzelnen Leiter gebildet, der sich gerade durch den Ferritkern hindurch erstreckt und endseitig jeweils mit den unteren Abschnitten der Klemmarme 441 des Klemmteils 44 verschraubt ist (Schraube 901). Die Klemmarme 441 sind elektrisch leitend und bilden Teile des Sekundärkreises, der durch das ebenfalls elektrisch leitende Schweissmesser 4 geschlossen wird, sobald dieses zwischen dem ersten 43 und dem zweiten Klemmteil 44 eingeklemmt und mit den Klemmarmen 441 in Kontakt ist. Da das Schweissmesser 4 gegenüber dem die Sekundärwicklung bildenden geraden Leiter und den beiden Klemmarmen 441 einen wesentlich geringeren Querschnitt und einen höheren spezifischen Widerstand aufweist, ist sein elektrischer Widerstand vergleichsweise hoch, so dass in ihm der Grossteil der elektrischen Verlustleistung des Sekundärkreises anfällt. Zudem ergibt sich durch das Übersetzungsverhältnis der Transformatoreinheit 90 mit der lediglich einen Windung im Sekundärkreis ein hoher Sekundärstrom, so dass das Schweissmesser sehr effektiv und schnell innerhalb von 3 bis 4 Sekunden auf die gewünschte Temperatur von ca. 300 °C aufgeheizt wird. Ströme grösser als 100 A können hierbei erreicht werden.

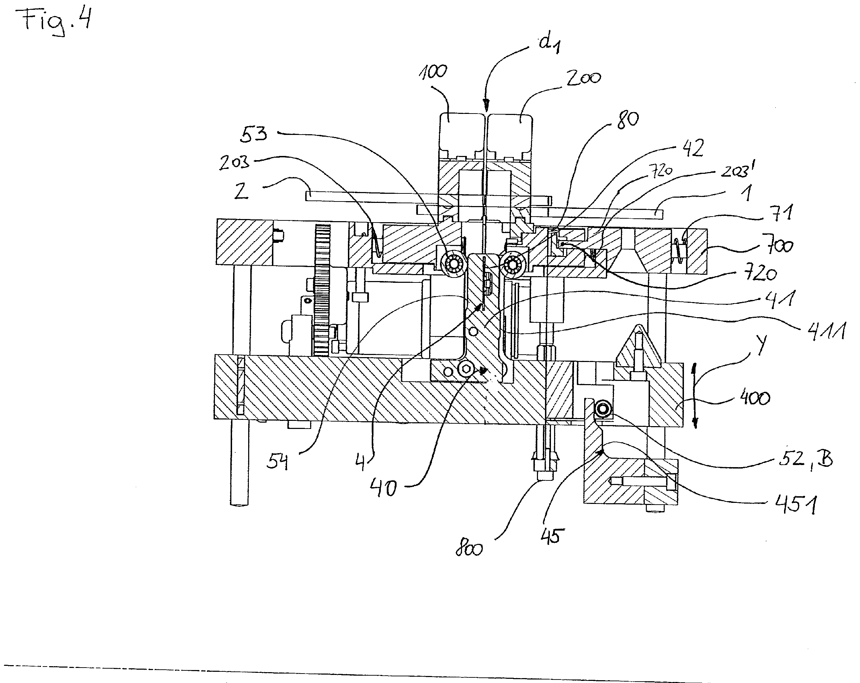

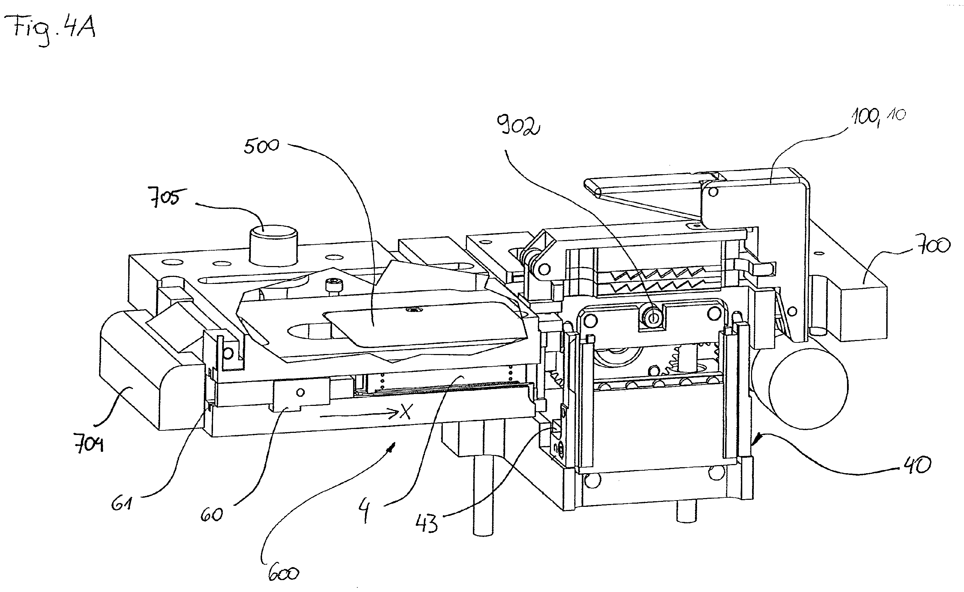

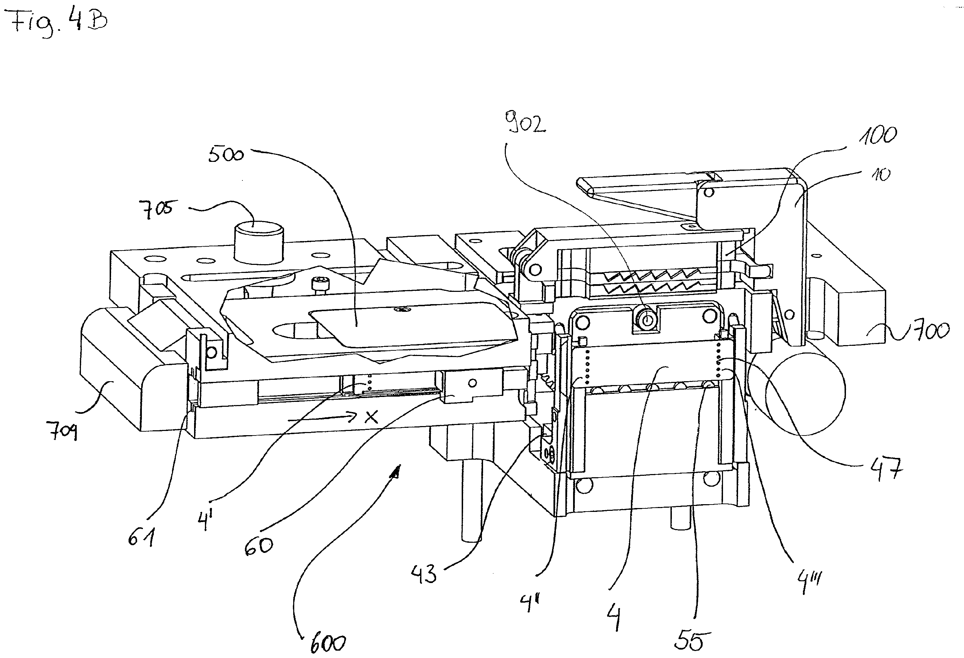

**Figur 4** zeigt eine Schnittdarstellung der Vorrichtung, wobei sich die Plattform 400 und mit ihr der Schweissmesserhalter 40, die ja in vertikaler Richtung des Pfeils Y zum Tisch 700 bewegbar ist, in der sogenannten "Bladefeed Position" befindet. In dieser Position befindet sich der Schweissmesserhalter 40 auf einer Höhe, so dass das Schweissmesser 4 durch den Schlitz 42 zwischen die beiden Klemmteile 43, 44 eingeschoben werden kann. Dazu sind die beiden Klemmteile 43, 44 etwas voneinander beabstandet. Der Abstand der beiden Klemmteile 43, 44 voneinander wird entgegen der Wirkung der Federn 51 durch Abtasten einer zweiten Kulisse 451 an einem zweiten Kulissenträger 45 mit Hilfe eines Abtastrades 52 eingestellt. In der "Bladefeed Position" befindet sich das Abtastrad 52 an der zweiten Kulisse 451 in einer Aufnahmeposition (B), in der die beiden Klemmteile 43, 44 gerade den für das Einschieben des Schweissmessers 40 nötigen Abstand voneinander haben.

Die beiden Schlauchhalter 100, 200 sind im Tisch 700 jeweils verschiebbar montiert und somit nicht lediglich gegeneinander, sondern auch in gleicher Richtung miteinander. Durch zweite Federn sind sie gegeneinander vorgespannt. Die entsprechenden zweiten Federn sind in Figur 4 mit 203, 203' bezeichnet. An den Schlauchhaltern 100, 200 ist weiter jeweils ein Paar von Abtasträdern 53 gelagert. Die dadurch insgesamt vier Abtasträder 53 wirken mit den erwähnten vier ersten Kulissen 411 an den Kulissenträgern 41 zusammen, wenn diese, wie in der in Figur 4 dargestellten "Bladefeed Position", zwischen die Abtasträderpaare 53 eingreifen. Durch das Abtasten der vier ersten Kulissen 411 wird der horizontale Abstand d1 zwischen den beiden Schlauchhaltern 100, 200 eingestellt. In der in Figur 4 dargestellten "Bladefeed Position" befinden sich der erste und der zweite Schlauchhalter 100, 200 im Abstand d1 zu einander. Indem die vier ersten Kulissen 411 identisch und spiegelsymmetrisch zur Ebene des Schweissmessers 4 ausgebildet sind und die beiden Schlauchhalter 100, 200 in gleicher Richtung miteinander verschiebbar sind, werden die Schlauchhalter 100, 200 relativ zum Schweissmesserhalter 40 stets so ausgerichtet, dass das Schweissmesser sich genau in der Mitte zwischen den beiden Schlauchhaltern 100, 200 befindet. Allfällige Toleranzen zwischen der Plattform 400, die den Schweissmesserhalter 40 trägt, und dem Tisch 700, auf dem die Schlauchhalter 100, 200 montiert sind, werden dadurch ausgeglichen.

**Figur 4A** zeigt einen Teil der Vorrichtung. Dargestellt wird einer der beiden Schlauchhalter 100 mit dem Spannhebel 10, angeordnet am Tisch 700. Die zweite Hälfte der Vorrichtung, umfassend den zweiten Schlauchhalter 200 und das zweite Klemmteil 44, ist in Figur 4A weggelassen. Teilweise sichtbar ist dadurch der Schweissmesserhalter 40 mit dem ersten Klemmteil 43. Der Schweissmesserhalter 40 befindet sich, wie in Fig. 4 beschrieben, in der "Bladefeed Position". Die Schläuche der ersten und zweiten Schlauchgruppe 1, 2 sollten in dieser Position an sich bereits in die Vorrichtung eingelegt sein, wobei sie in Figur 4 jedoch ebenfalls weggelassen sind. Die Schweissmesserzuführung 600 umfasst den Schieber 60, welcher mit dem Schlitten 601 entlang von Führungsschienen 61 bewegt und mit Hilfe eines weiteren Antriebs 602 angetrieben wird (vgl. Figur 1A). Der Schieber 60 wird zur Einführung des Schweissmessers 4 in den Schweissmesserhalter 40 in Richtung des Pfeils X bewegt und nimmt dabei das Schweissmesser 4 mit. Pro Schweissvorgang wird ein neues Schweissmesser eingeführt, sodass jedes Schweissmesser 4 nur einmal benutzt wird. Eine grössere Anzahl an Schweissmessern 4 ist in der Box 500 bevorratet. Die Box 500 ist derart ausgestaltet, dass pro Schweissvorgang jeweils ein Schweissmesser 4 in den Bereich der Führungsschienen 61 vorgeschoben wird, so dass es durch den Schieber 60 aufgegriffen und in den Schweissmesserhalter 40 verschoben werden kann. Ebenfalls in Fig. 4A ersichtlich ist ein Temperatursensor 902, mit welchem die Temperatur des Schweissmessers erfasst und kontrolliert wird.

**Figur 4B** zeigt die Teildarstellung aus Figur 4A, wobei das Schweissmesser 4 in den Schweissmesserhalter 40 eingeschoben ist. Ein weiteres Schweissmesser 4' ist für den nächsten Schweissvorgang bereits vorpositioniert. Im Schweissmesserhalter 40 wird das Schweissmesser 4 auf halbmondförmigen Schienen 55 geführt. Zum vollständigen Einführen des Schweissmessers 4 greift der Schieber 60 selbst ein Stück weit in den Schweissmesserhalter 40 ein. Bevor die weiteren Schritte mit vertikaler Verschiebung des Schweissmesserhalters 40 aus der "Bladefeed Position" ausgeführt werden können, muss der Schieber 60 daher unter Umkehr seiner Bewegungsrichtung zunächst aus dem Bereich des Schweissmesserhalters 40 wieder herausgefahren werden. Figur 4B zeigt den Schieber 60 bereits schon ein Stück weit wieder zurückgefahren.

Figur 4B zeigt das Schweissmesser 4 in einer bevorzugten Ausführungsform. Entlang seiner beiden kürzeren Seiten des Rechtecks verlaufen Randbereiche 4" und 4‴. Im eingesetzten Zustand werden diese Randbereiche 4" und 4‴ durch das erste und das zweite Klemmteil 43, 44 kontaktiert (nicht sichtbar). Für eine gute elektrische Kontaktierung weist das Klemmteil 44 an der Innenseite der aufragenden Abschnitte der Klemmarme 441 hierzu vorspringende Kontaktpunkte, vorzugsweise in Form federnder Druckstücke, auf. In der Nähe der Randbereiche weist das Schweissmesser 4 Ausnehmungen 48 auf, welche als Bohrungen entlang der beiden kurzen Rechteckseiten ausgestaltet sind. Entlang seiner unteren Rechteckseite liegt das Schweissmesser 4 punktuell auf den halbmondförmigen Schienen 55 auf.

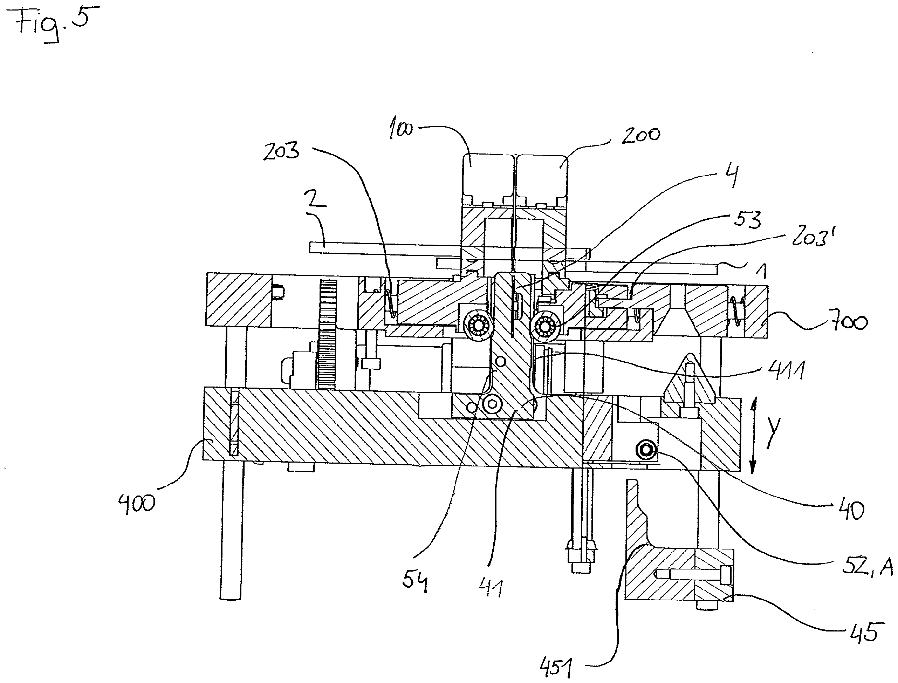

**Figur 5** zeigt eine Schnittdarstellung der Vorrichtung, wobei sich die Plattform 400, mit Hilfe derer der Schweissmesserhalter 40 in vertikaler Richtung des Pfeils Y bewegt wird, in der sogenannten "Pre-cut Position" befindet. Gegenüber und ausgehend von der "Bladefeed Position" gemäss Figur 4 ist die Plattform 400 ein Stück weit nach oben verschoben. In dieser Position befindet sich nun das Abtastrad 52 oberhalb des zweiten Kulissenträgers 45 ausser Eingriff mit der zweiten Kulisse 451 in einer sogenannten Kontaktierungsposition (A). In dieser Position werden die beiden Klemmteile 43, 44 nicht länger durch die zweite Kulisse 451 auf Abstand voneinander gehalten, sondern können nunmehr das Schweissmesser 4 zwischen sich einklemmen, wobei es gleichzeitig elektrisch kontaktiert wird. In dieser Position erfolgt dann auch die elektrische Beheizung des Schweissmessers 4.

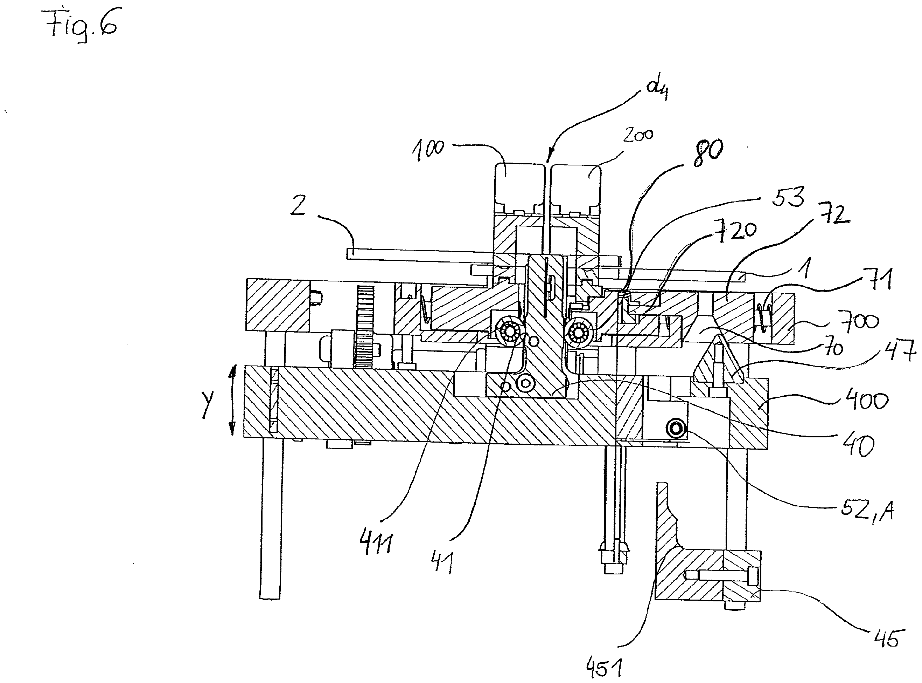

**Figur 6** zeigt eine Schnittdarstellung der Vorrichtung, wobei sich die Plattform 400, mit Hilfe derer der Schweissmesserhalter 40 in vertikaler Richtung des Pfeils Y bewegt wird, in der sogenannten "Stretch Position" befindet. Verglichen mit Figur 4 und Figur 5 wurde die Plattform 400 weiter in Richtung des Tisches 700 bewegt und die Abtasträder 53 befinden sich nunmehr an den ersten Kulissen 411 in einer Position, in der der Abstand der beiden Schlauchhalter 100, 200 den grössten Wert d4 annimmt. Die Schläuche der ersten und der zweiten Schlauchgruppe 1, 2 werden dadurch gedehnt. Die ersten Kulissen 411 angeordnet an den Kulissenträgern 41 sind so ausgebildet, dass dieser Abstand d4 bei der folgenden Weiterbewegung der Plattform 400 in Richtung des Tisches 700, während der das Schneidmesser 4 die Schläuche durchtrennt, erhalten bleibt. Die Schläuche werden dadurch im gedehnten Zustand durchtrennt.

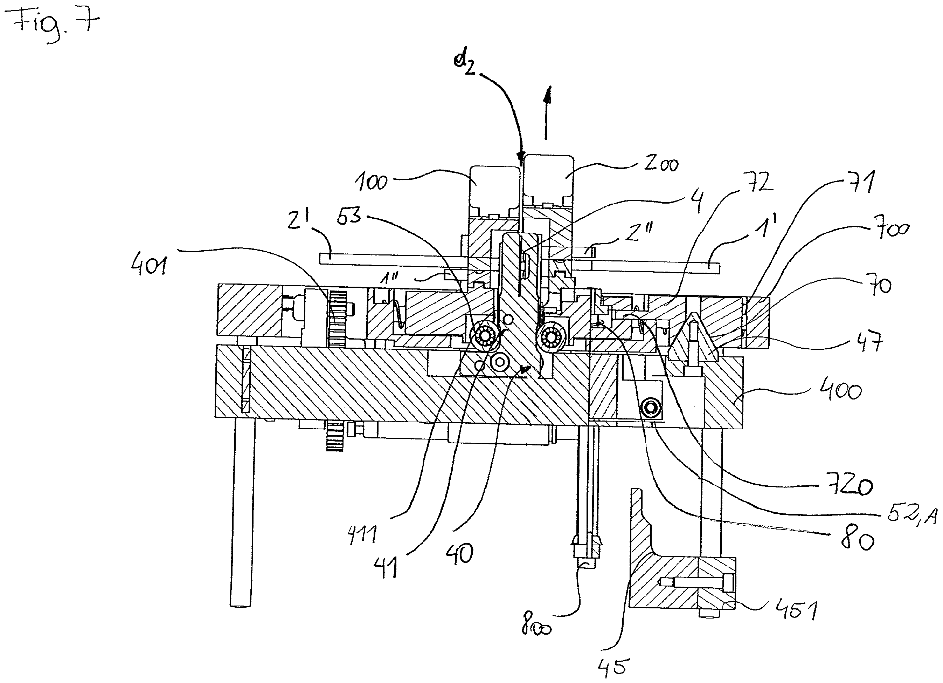

**Figur 7** zeigt eine Schnittdarstellung der Vorrichtung, wobei sich die Plattform 400 in der sogenannten "Cut Position" befindet, in welcher die Schläuche der ersten und der zweiten Schlauchgruppe 1, 2 durchtrennt sind.

Unmittelbar nach dem Durchtrennen der Schläuche müssen die Schnitt-Enden 1' der Schläuche der ersten Schlauchgruppe auf die Schnitt-Enden 2' der Schläuche der zweiten Schlauchgruppe in der Höhe ausgerichtet werden. Dazu ist der zweite Schlauchhalter 200 vertikal verschiebbar und mittels einer dritten Feder 80 nach oben vorgespannt. Diese Bewegung des zweiten Schlauchhalters 200 ist in den soweit beschriebenen Positionen jedoch blockiert durch den Eingriff eines Anschlagsfläche 720 an einem Riegel 72, der im Tisch 700 horizontal verschiebbar und von einer vierten Feder 71 gegen den zweiten Schlauchhalter vorgespannt ist. Die Entriegelung des zweiten Schlauchhalters 200 durch Zurückziehen der Anschlagsfläche 720 erfolgt durch Eingriff eines an der Plattform 400 angebrachten Kegels 47 in einen exzentrisch zu diesem angeordneten Hohlkegel 70 auf der Unterseite des Riegels 72. Die vierte Feder 71 wird dabei zusammengedrückt. Wie dies in Figur 6 bereits erkennbar ist, beginnt sich in der "Stretch Position" der Kegel 47 bereits in den Hohlkegel 70 einzuschieben. Der Kegel 47 und der Hohlkegel 70 sind so aufeinander abgestimmt, dass die Verriegelung des zweiten Schlauchhalters 200 bei der vertikalen Bewegung der Plattform 400 gelöst wird, sobald die Schläuche beider Schlauchgruppen 1, 2 vollständig durchtrennt sind. Nach dem Lösen der Verriegelung drückt die dritte Feder 80 den zweiten Schlauchhalter 200 nach oben.

Wie in Figur 7 dargestellt, greift in der "Cut-Position" der Kegel 47 vollständig in den Hohlkegel 70 ein und hat die Verriegelung des zweiten Schlauchhalters 200 gelöst, so dass dieser durch die dritte Feder 80 nach oben verschoben werden konnte. Dadurch sind die Schnitt-Enden 1' der Schläuche der ersten Schlauchgruppe auf die Schnitt-Enden 2' der Schläuche der zweiten Schlauchgruppe in der Höhe ausgerichtet. Das Schweissmesser 4 befindet sich noch zwischen den Schläuchen.

In der "Cut Position" hat die Plattform 400 ihre höchste Position erreicht. Das Abtastrad 52 der zweiten Kulisse 451 befindet sich weiterhin oberhalb von dieser in der Kontaktierungsposition (A), so dass das Schweissmesser 4 zwischen den Klemmteilen 43, 44 eingeklemmt ist und beheizt werden kann und auch wird. Über die Abtasträder 53 wird an den ersten Kulissen 411 der Abstand der Schlauchhalter 100, 200 zu einander auf den Abstand d2 eingestellt, der so bemessen ist, dass die Schnitt-Enden 1' und 2' sowie die Rest-Enden 1", 2" an das heisse Schweissmesser 4 gedrückt und dadurch aufgeschmolzen werden.

Danach wird das Schweissmesser 4 aus dem Bereich der geschnittenen Schläuche herausgefahren. Dies erfolgt, indem die Plattform 400 und mit ihr der Schweissmesserhalter 40 nunmehr nach unten bewegt wird. Die Umkehr ihrer Bewegungsrichtung erfolgt ohne Umkehr der Drehrichtung des Motors 404 des Antriebs 401 automatisch durch das Pleuel 405. Dabei passieren die Abtasträder 53 an den ersten Kulissen 411 zunächst wieder den Bereich, den sie während der "Stretch Position" bereits überfahren haben, während dessen der Abstand der beiden Schlauchhalter 100, 200 den grösseren Wert d4 aufweist. Die Schläuche mit ihren aufgeschmolzenen Enden werden dadurch vom Schweissmesser 4 etwas wegbewegt, was dessen Herausfahren erleichtert. Ferner wird kein Schmelzmaterial, welches sich an den aufgeschmolzenen Enden befindet durch das Herausfahren abtransportiert und steht für das anschliessende Verschweissen zur Verfügung.

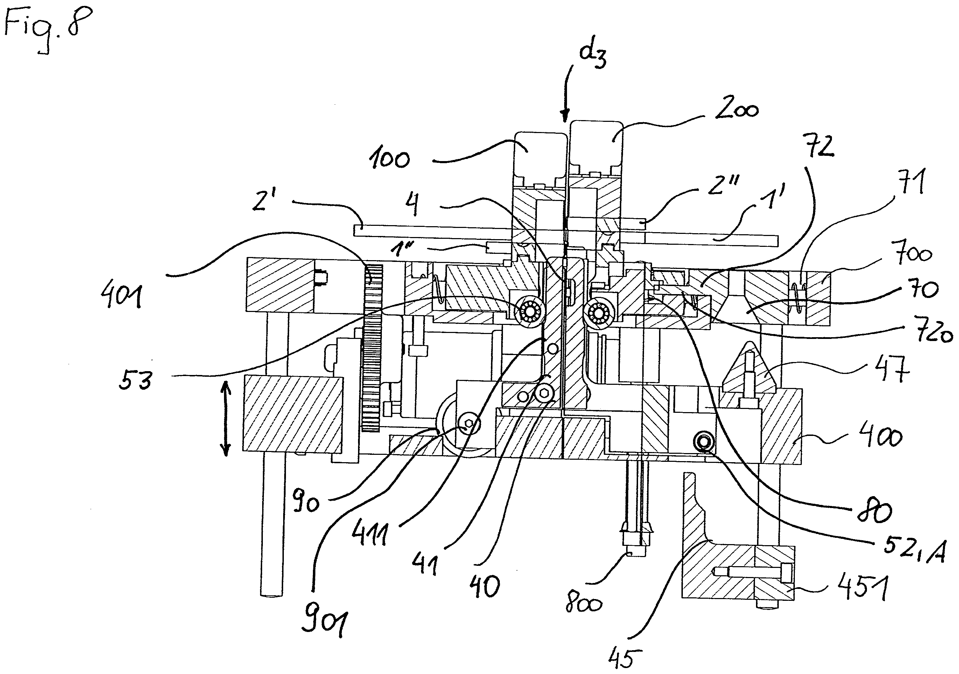

Bei der Weiterbewegung der Plattform 400 nach unten kommen die Abtasträder 53 an den ersten Kulissen 411 wieder in den Bereich, den sie während der "Precut Position" überfahren haben und während dem der Abstand der beiden Schlauchhalter 100, 200 den kleineren Wert d3 aufweist. Hierdurch kommt je ein Schnitt-Ende 1' der ersten Schlauchgruppe mit je einem Schnitt-Ende 2' der zweiten Schlauchgruppe in Berührung und wird zu durchgängigen Schläuchen verschweisst. Diese sogenannte "Press-Position" zeigt **Figur 8****.** In der "Press-Position von Fig. 8 bleibt die Plattform 400 stehen, so dass die gefügten Schläuche sowie die Rest-Enden 1", 2", nach einer Abkühlung, aus der Vorrichtung entnommen werden können.

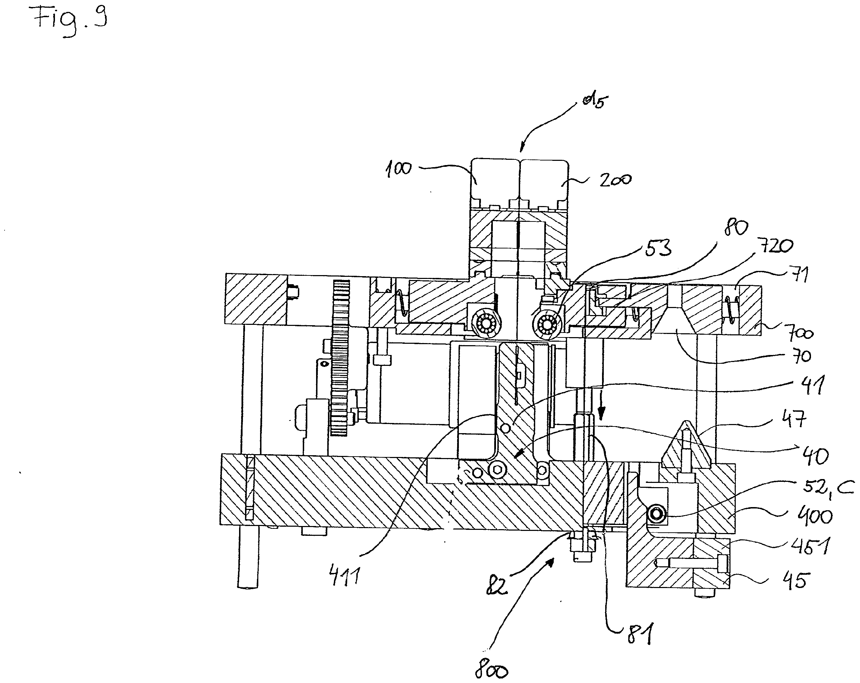

**Figur 9** zeigt eine Schnittdarstellung der Vorrichtung, mit der Plattform 400 und dem Schweissmesserhalter 40 in der "Eject Position". In der "Eject Position" befindet sich die Plattform 400 in ihrer tiefsten Position. Die verschweissten Schläuche und die Rest-Enden wurden bereits aus den Schlauchhaltern 100, 200 entnommen. Wie bereits zu Figur 4 erwähnt, wird der Abstand der beiden Klemmteile 43, 44 zu einander durch Abtasten der zweiten Kulisse 451 mit Hilfe eines Abtastrades 52 eingestellt. Ist der Schweissmesserhalter 40 in der "Eject Position", so befindet sich das Abtastrad 52 der zweiten Kulisse 451 in einer Freigabeposition (C). Das erste Klemmteil 43 wurde dabei entgegen der Wirkung der ersten Federn 51 (in Figur 9 nicht sichtbar) relativ zum zweiten Klemmteil 44 so bewegt, sodass die Spaltbreite zwischen den beiden Klemmteilen 43, 44 auf maximale Distanz geöffnet ist. Das Schweissmesser 4 ist dann frei und kann aus dem Schweissmesserhalter 40 in eine Entsorgungsbox (in Figur 9 nicht dargestellt) nach unten herausfallen.

Beim Übergang von der "Press Position" gemäss Fig. 8 in die "Eject Position" gemäss Fig. 9 wird der zweite Schlauchhalter 200 aus seiner nach oben verschobenen Stellung in seine Ausgangsposition auf gleicher Höhe mit dem ersten Schlauchhalter 100 zurückbewegt. Gleichzeitig wird die dritte Feder 80 gespannt. Dazu dient ein Rückholmechanismus 800 mit einer mit dem zweiten Schlauchhalter 200 gekoppelten Zugstange 81.

Beim nach unten Fahren schlägt die Plattform 400 an einem Anschlag 82 der Zugstange 81 an und zieht diese mit nach unten. Auch der Kegel 47 kommt dabei ausser Eingriff mit dem Hohlkegel 70, so dass die Anschlagsfläche 720 wie zuvor in den zweiten Schlauchhalter 200 einrasten. Der zweite Schlauchhalter befindet sich in seiner Ausgangsposition auf gleicher Höhe mit dem ersten Schlauchhalter 100.

Beim nach unten Fahren in die "Eject Position" kommen schliesslich die Abtasträder 53 ebenfalls ausser Eingriff Kontakt mit den ersten Kulissen 411 an den Kulissenträgern 41. Der Abstand zwischen den beiden Schlauchhaltern 100, 200 verringert sich dabei soweit, dass die Schlauchhalter 100, 200 einander berühren.

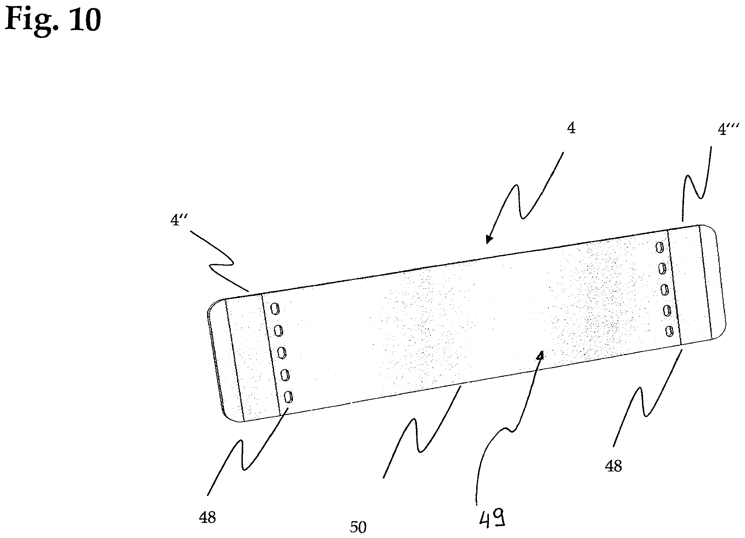

**Figur 10** zeigt die bevorzugte Ausführungsform des Schweissmessers 4 in einer rechteckigen Form mit Ausnehmungen 48, angeordnet jeweils in einer Reihe entlang der Randbereiche 4", 4‴ der beiden kürzeren Rechtecksseiten. Das Schweissmesser 4 ist vorzugsweise durch hälftige Längsfaltung eines Blättchens von doppelter Breite hergestellt. Als Klinge 50, welche die Schläuche schneidet, dient die Biegekante entlang von welcher die beiden Hälften des Blättchens zusammenhängen. Alternativ zur Faltung wird das Schweissmesser 4 durch Stanzen und dessen Klinge 50 durch Prägung hergestellt. Durch diese Gestaltung wird in rationeller Weise eine nicht zu scharfe, sondern abgerundete, vor allem jedoch gleichmässige und zu beiden Seiten hin symmetrische Schneidkante geschaffen. Letzteres ist wichtig, damit das Schneidmesser beim Durchtrennen der Schläuche nicht zu einer Seite hin abgelenkt wird, wie dies beispielsweise der Fall bei einer unsymmetrisch geschliffenen Kante wäre.

Die Klinge 50, welche die Schläuche schneidet, verläuft entlang einer der beiden längeren Rechtecksseiten.

Das Schweissmesser 4 weist beispielsweise eine Länge zwischen 60-80 mm, insbesondere von 70 mm, eine Breite zwischen 15-20 mm, insbesondere von 17 mm und eine Stärke zwischen 0.2-0.5 mm, insbesondere von 0.3 mm auf. Die Grösse der Schmelzfläche 49 hängt in erster Linie vom Durchmesser der Schläuche und von der Anzahl der zu verschweissenden Schläuche ab.

Beim verwendeten Material für das Schweissmesser 4 handelt es sich beispielsweise um Chrom/Nickelstahl.

Für ein qualitativ hochwertiges Schweissergebnis relevant ist, die horizontale sowie die vertikale Temperaturverteilung über die Fläche des Schweissmessers. Es wurde festgestellt, dass die Anordnung der Ausnehmungen entlang der Randbereiche 4", 4‴ zu einer homogeneren vertikalen Wärmeverteilung führt. Die gewünschte Temperatur des Schweissmessers beträgt ca. 300 °C.

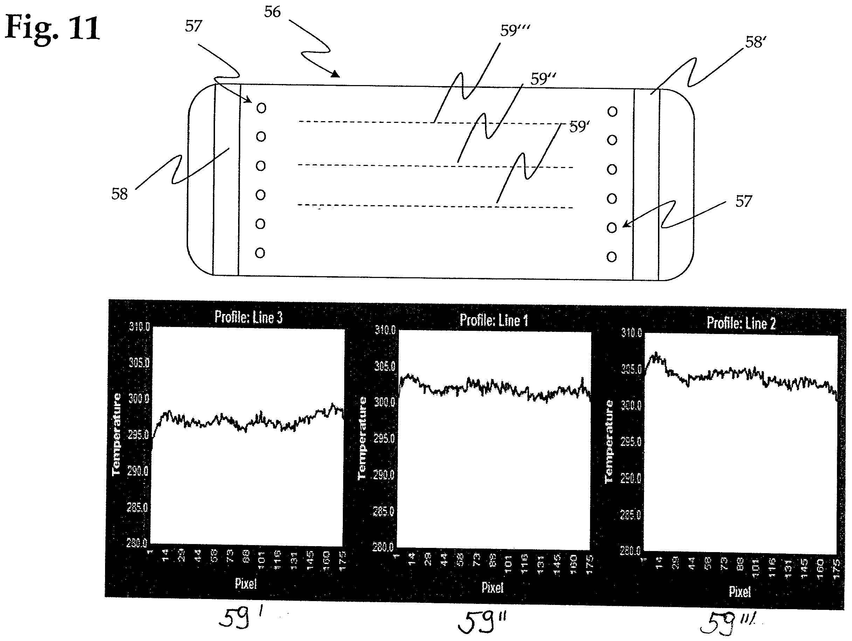

**Figur 11** zeigt eine weitere Ausführungsform eines Schweissmessers 56. Hier sind pro Randzone 58, 58' beispielsweise je sechs, vorzugsweise kreisrunde Ausnehmungen 57 angeordnet. Die Anzahl sowie die geometrische Form dieser Ausnehmung ist nicht auf die dargestellte Anzahl und eine kreisrunde Form beschränkt.

Das Verhältnis zwischen Stegbreite zu Schlitzbreite bzw. Durchmesser beträgt in der gezeigten Ausführungsform 60 %. Die darunterliegende Darstellung zeigt das Messprotokoll mit der horizontalen Temperaturverteilung entlang von drei strichlierten Linien 59', 59", 59‴. Abgelesen aus den Messprotokollen kann ebenfalls die vertikale Temperaturverteilung werden. Die Linien 59', 59", 59‴ wurden in einer Höhe gewählt, in welcher in der Vorrichtung später die Schläuche durchlaufen werden.

Dem Protokoll liegen Aufnahmen mit einer Wärmebildkamera zugrunde. In diesem Protokoll wird die horizontale Temperaturverteilung entlang der Linien 59', 59", 59‴ gezeigt. Das Profil "Linie 3" entspricht der Messung entlang der Linie 59'. Das Profil "Linie 1", jenem entlang der Linie 59" und das Profil "Linie 2" jenem entlang der Linie 59‴. Die auf der y-Achse angegebenen Werte der Pixel entsprechen horizontalen Längeneinheiten entlang der Linien 59', 59", 59‴.

Das Schweissmesser 56 wurde für die Messung der Temperaturverteilung in den Schweissmesserhalter 40 umfassend das erste und das zweite Klemmteil 43,44 eingespannt und für die Messung geschwärzt. Das Schweissmesser schliesst den Sekundärstromkreis der Transformatoreinheit und wird bedingt durch seinen hohen Widerstandswert auf eine Solltemperatur von ca. 300 °C erhitzt. Es konnte festgestellt werden, dass die optimale Temperaturverteilung (300 °C +/- 5 °C) innert 3-4 Sekunden erreicht war. Bei den Versuchen konnte des Weiteren herausgefunden werden, dass die Kontaktierung des Schweissmessers durch die beiden Klemmteile einen Einfluss auf die vertikale Temperaturverteilung hat. Eine symmetrische Verteilung der Klemmkraft entlang der beiden Randzonen hat sich als relevant für die vertikale Temperaturverteilung erwiesen. Eine Möglichkeit hierfür sind vorzugsweise federnde Druckstücke angeordnet auf mindestens einem der beiden Klemmteile. Mit Hilfe dieser Druckstücke wird eine symmetrische Kontaktierung erzielt, indem die Druckkraft aller Druckstücke in etwa gleich eingestellt wird.

### BEZEICHNUNGSLISTE

1 Schläuche der ersten Schlauchgruppe
1' Schnitt-Enden der Schläuche der ersten Schlauchgruppe
1" Rest-Enden der Schläuche der ersten Schlauchgruppe
2 Schläuche der zweiten Schlauchgruppe
2' Schnitt-Enden der Schläuche der zweiten Schlauchgruppe
2" Rest-Enden der Schläuche der zweiten Schlauchgruppe
100 Erster Schlauchhalter
101 erste Schlauchklemme des ersten Schlauchhalters
102 zweite Schlauchklemme des ersten Schlauchhalters
51 erste Feder
11 Durchgangsöffnungen durch erste Schlauchklemme zweiter Schlauchhalter
12 Durchgangsöffnungen durch zweite Schlauchklemme erster Schlauchhalter
200 zweiter Schlauchhalter
201 erste Schlauchklemme des zweiten Schlauchhalters
202 zweite Schlauchklemme des zweiten Schlauchhalters
203, 203' zweite Federn
21 Durchgangsöffnungen durch erste Schlauchklemme zweiten Schlauchhalters
22 Durchgangsöffnungen durch zweite Schlauchklemme zweiten Schlauchhalters
31 erste Quetschzone
32 zweite Quetschzone
4 Schweissmesser
4' weiteres Schweissmesser
4", 4‴ Randbereiche
40 Schweissmesserhalterung
41 ein Paar von Kulissenträgern
42 Schlitz
43 erstes Klemmteil
44 zweites Klemmteil
45 zweiter Kulissenträger
47 Kegel
48 Ausnehmungen
49 Schmelzfläche
50 Klinge
51 erste Federn
52 Abtastrad der zweiten Kulisse
53 Abtastradpaar der ersten Kulisse
55 halbmondförmige Schienen
56 Schweissmesser
57 kreisrunde Ausnehmungen
58, 58' Randzone
59', 59", 59‴ drei strichlierten Linien
400 Plattform zur Positionierung der Schweissmesserhalterung
401 Antrieb für die Plattform
402, 403, 407 Zahnräder
404 Motor
405 Pleuel
406 stangenförmige Führung
411 vier erste Kulissen
431, 441 abgewinkelte Klemmarme
451 zweite Kulisse
500 Box mit Schweissmessern
520 stangenförmige Führung
600 Schweissmesserzuführung
60 Schieber
61 Führungsschienen
601 Schlitten
602 Antrieb für Schweissmesserzuführung
603 Zahnriemen
700 Tisch
70 Hohlkegel
71 vierte Federn
72 Riegel
720 Anschlagsfläche
701 vier Beine
702 Ausschnitt
704 Schublade
705 Startknopf
10, 20 Spannhebel
800 Rückholmechanismus
80 dritte Feder
81 Zugstange
82 Anschlag
90 Transformatoreinheit
901 Schraube
902 Temperatursensor
Kontaktierungsposition (A)
Aufnahmeposition (B)
Freigabeposition (C)

## Patentansprüche

1. Schweissmesser (4, 4', 56) für eine Vorrichtung zum Verschweissen von thermoplastischen Schläuchen, wobei das Schweissmesser (4, 4', 56) oder eine Schmelzfläche (49) des Schweissmessers (4, 4', 56) eine rechteckige Form hat, an einer der beiden längeren Seiten des Rechtecks eine Klinge (50) aufweist, elektrisch leitend ist, die Schmelzfläche eine Vielzahl von Ausnehmungen (48) aufweist **dadurch gekennzeichnet, dass** entlang der beiden kürzeren Seiten des Rechtecks Randzonen (4", 4‴) vorgesehen sind, welche dazu eingerichtet sind, zur elektrischen Beheizung des Schweissmessers (4, 4', 56) durch elektrische Verlustleitung im Schweissmesser (4, 4', 56) kontaktiert zu werden, wobei die Ausnehmungen (48, 57), jeweils in einer Reihe entlang der Randzonen (4", 4") der beiden kürzeren Seiten des Rechtecks vorgesehen sind.

2. Schweissmesser (4, 4', 56) nach Anspruch 1, **dadurch gekennzeichnet, dass** die Ausnehmungen (57) hinsichtlich ihrer geometrischen Form kreisrund sind.

3. Schweissmesser (4, 4', 56) nach Anspruch 1 oder 2, **dadurch gekennzeichnet, dass** die Ausnehmungen als Bohrungen (48, 57) ausgestaltet sind.

4. Schweissmesser (4, 4', 56) nach einem der vorangegangenen Ansprüche, **dadurch gekennzeichnet, dass** pro Randzone sechs Ausnehmungen (48, 57) vorgesehen sind.

5. Schweissmesser (4, 4', 56) nach einem der vorangegangenen Ansprüche, **dadurch gekennzeichnet, dass** das bezogen auf die Ausnehmungen (48, 57) entlang der Randzonen (4", 4‴) der beiden kürzeren Seiten des Rechtecks bestimmte Verhältnis zwischen Stegbreite zu Schlitzbreite bzw. Durchmesser, 60% beträgt.

6. Schweissmesser (4, 4', 56) nach einem der vorangegangenen Ansprüche, **dadurch gekennzeichnet, dass** die Randzonen (4", 4‴) für eine Kontaktierung durch Einklemmen mit symmetrischer Verteilung der Klemmkraft entlang der Randzonen (4', 4") geeignet ausgebildet sind.

7. Schweissmesser (4, 4', 56) nach einem der vorangegangen Ansprüche, **dadurch gekennzeichnet, dass** das Schweissmesser durch hälftige Längsfaltung eines Blättchens von doppelter Breite hergestellt ist, wobei als Klinge (50) die Biegekante dient, entlang von welcher die beiden Hälften des Blättchens zusammenhängen.

8. Schweissmesser (4, 4', 56) nach einem der vorangegangen Ansprüche 1 bis 6, **dadurch gekennzeichnet, dass** das Schweissmesser (4, 4', 56) durch Stanzen und die Klinge (50) durch Prägung hergestellt ist, wobei die Prägung entlang der Längsseite des Rechtecks eingepresst wird.

9. Schweissmesser (4, 4', 56) nach Anspruch 8, **dadurch gekennzeichnet, dass** die Klinge (50) eine nicht zu scharfe, sondern abgerundete, gleichmässige und zu beiden Seiten symmetrisch ausgebildete Schneidkante aufweist.

10. Box (500) umfassend eine Vielzahl von darin bevorrateten Schweissmessern (4, 4', 56) nach einem der Ansprüche 1 - 9.

11. Box (500) nach Anspruch 10, **dadurch gekennzeichnet, dass** die einzelnen Schweissmesser (4, 4', 56) in der Box (500) in Längsrichtung aufgestellt einsortiert sind.

12. Box (500) Anspruch 10 oder 11, **dadurch gekennzeichnet, dass** die Box einseitig offen ist und dass ein in der Box angeordnetes Federelement das für nächsten Schweissvorgang vorgesehene Schweissmesser in Richtung der einseitigen Öffnung drückt.

13. Box (500) nach einem der Ansprüche 10 - 12, **dadurch gekennzeichnet, dass** die Box (500) als Ganzes austauschbar ist.

14. Schweissmesser (4, 4', 56) nach einem der vorangegangenen Ansprüche 1 bis 9, **dadurch gekennzeichnet, dass** das Schweissmesser (4, 4', 56) in zwei sich gegenüberliegenden Klemmteile (43), (44) eines Schweissmesserhalters (40) einklemmbar ist.

15. Schweissmesser (4, 4', 56) nach Anspruch 14, **dadurch gekennzeichnet, dass** das Schweissmesser (4) elektrisch kontaktierbar ist.

16. Schweissmesser (4, 4', 56), nach Anspruch 14 oder 15, **dadurch gekennzeichnet, dass** das Schweissmesser (4, 4', 56) mit einem der beiden Klemmteile (43, 44) des Schweissmesserhalters (40) mit einer aus einem einzigen Leiter gebildeten Sekundärwicklung einer Transformatoreinheit (90) verbindbar ist und das Schweissmesser (4) den Sekundärstromkreis der Transformatoreinheit (90) schliesst.

17. Schweissmesser (4, 4', 56), nach einem der Ansprüche 14 bis 16, **dadurch gekennzeichnet, dass** das Schweissmesser (4, 4', 56) durch Durchstücke, federnd gelagert angeordnet an mindestens einem der beiden Klemmteile (43), (44), kontaktierbar ist.

## Claims

1. Welding knife (4, 4', 56) for a device for welding thermoplastic tubes, wherein the welding knife (4, 4', 56) or a melting surface (49) of the welding knife (4, 4', 56) has a rectangular shape, has a blade (50) on one of the two longer sides of the rectangle, is electrically conducting, the melting surface has a plurality of recesses (48), **characterized in that** provided along the two shorter sides of the rectangle are peripheral zones (4", 4‴) which are configured to be contacted for electrically heating the welding knife (4, 4', 56) by an electrical power loss in the welding knife (4, 4', 56), wherein the recesses (48, 57) are provided in a row along the peripheral zones (4", 4") of the two shorter sides of the rectangle, respectively.

2. Welding knife (4, 4', 56) according to Claim 1, **characterized in that** the recesses (57) in terms of the geometric shape thereof are circular.

3. Welding knife (4, 4', 56) according to Claim 1 or 2, **characterized in that** the recesses are provided as bores (48, 57).

4. Welding knife (4, 4', 56) according to one of the preceding claims, **characterized in that** six recesses (48, 57) are provided for each peripheral zone.

5. Welding knife (4, 4', 56) according to one of the preceding claims, **characterized in that** the ratio between the web width and slot width, or diameter, respectively, determined with respect to the recesses (48, 57) along the peripheral zones (4", 4‴) of the two shorter sides of the rectangle, is 60%.

6. Welding knife (4, 4', 56) according to one of the preceding claims, **characterized in that** the peripheral zones (4", 4‴) are configured to be suitable for contacting by clamping with a symmetrical distribution of the clamping force along the peripheral zones (4', 4").

7. Welding knife (4, 4', 56) according to one of the preceding claims, **characterized in that** the welding knife is produced by longitudinally folding a lamella of twice the width in half, wherein the bending edge along which the two halves of the lamella are connected serves as the blade (50).

8. Welding knife (4, 4', 56) according to one of preceding Claims 1 to 6, **characterized in that** the welding knife (4, 4', 56) is produced by punching, and the blade (50) is produced by stamping, wherein the stamping is impressed along the longitudinal side of the rectangle.

9. Welding knife (4, 4', 56) according to Claim 8, **characterized in that** the blade (50) has a uniform cutting edge which is not too sharp but rounded, and is configured to be symmetrical on both sides.

10. Box (500) comprising a plurality of welding knifes (4, 4', 56) according to one of Claims 1 to 9 stored therein.

11. Box (500) according to Claim 10, **characterized in that** the individual welding knifes (4, 4', 56) are sorted so as to be set up in the longitudinal direction in the box (500).

12. Box (500) according to Claim 10 or 11, **characterized in that** the box is open on one side, and **in that** a spring element disposed in the box presses the welding knife provided for the next welding procedure in the direction of the unilateral opening.

13. Box (500) according to one of Claims 10 to 12, **characterized in that** the box (500) can be exchanged as a whole.

14. Welding knife (4, 4', 56) according to one of preceding claims 1 to 9, **characterized in that** the welding knife (4, 4', 56) is able to be clamped in two mutually opposite clamping parts (43, 44) of a welding knife holder (40).

15. Welding knife (4, 4', 56) according to Claim 14, **characterized in that** the welding knife (4) is electrically contactable.

16. Welding knife (4, 4', 56) according to Claim 14 or 15, **characterized in that** the welding knife (4, 4', 56) by way of one of the two clamping parts (43, 44) of the welding knife holder (40) is able to be connected to a secondary winding of a transformer unit (90) that is formed by a single conductor, and the welding knife (4) closes the secondary current circuit of the transformer unit (90).

17. Welding knife (4, 4', 56) according to one of Claims 14 to 16, **characterized in that** the welding knife (4, 4', 56) is contactable by pressure pads which are disposed so as to be spring-mounted on at least one of the two clamping parts (43, 44).

## Revendications

1. Couteau de soudage (4, 4', 56) pour un dispositif de soudage de tuyaux thermoplastiques, le couteau de soudage (4, 4', 56) ou une surface de fusion (49) du couteau de soudage (4, 4', 56) ayant une forme rectangulaire, présentant une lame (50) sur l'un des deux côtés les plus longs du rectangle, étant électriquement conducteur, la surface de fusion présentant une pluralité d'évidements (48), **caractérisé en ce que** le long des deux côtés les plus courts du rectangle sont prévues des zones de bord (4", 4‴) qui sont configurées pour être mises en contact pour le chauffage électrique du couteau de soudage (4, 4', 56) par une puissance dissipée électrique dans le couteau de soudage (4, 4', 56), les évidements (48, 57) étant respectivement prévus en une rangée le long des zones de bord (4", 4") des deux côtés les plus courts du rectangle.

2. Couteau de soudage (4, 4', 56) selon la revendication 1, **caractérisé en ce que** les évidements (57) sont circulaires en ce qui concerne leur forme géométrique.

3. Couteau de soudage (4, 4', 56) selon la revendication 1 ou 2, **caractérisé en ce que** les évidements sont conçus sous forme d'alésages (48, 57).

4. Couteau de soudage (4, 4', 56) selon l'une quelconque des revendications précédentes, **caractérisé en ce que** six évidements (48, 57) sont prévus par zone de bord.

5. Couteau de soudage (4, 4', 56) selon l'une quelconque des revendications précédentes, **caractérisé en ce que** le rapport entre la largeur d'entretoise et la largeur de fente ou le diamètre, déterminé par rapport aux évidements (48, 57) le long des zones de bord (4", 4''') des deux côtés les plus courts du rectangle, est de 60 %.

6. Couteau de soudage (4, 4', 56) selon l'une quelconque des revendications précédentes, **caractérisé en ce que** les zones de bord (4", 4‴) sont configurées pour être appropriées pour un contact par serrage avec une répartition symétrique de la force de serrage le long des zones de bord (4', 4").

7. Couteau de soudage (4, 4', 56) selon l'une quelconque des revendications précédentes, **caractérisé en ce que** le couteau de soudage est fabriqué par pliage longitudinal à moitié d'une feuille de largeur double, l'arête de pliage, le long de laquelle les deux moitiés de la feuille sont jointes, servant de lame (50).

8. Couteau de soudage (4, 4', 56) selon l'une quelconque des revendications 1 à 6 précédentes, **caractérisé en ce que** le couteau de soudage (4, 4', 56) est fabriqué par estampage et la lame (50) par emboutissage, l'emboutissage étant pressé le long du côté longitudinal du rectangle.

9. Couteau de soudage (4, 4', 56) selon la revendication 8, **caractérisé en ce que** la lame (50) présente une arête de coupe non pas trop tranchante, mais arrondie, uniforme et symétrique par rapport aux deux côtés.

10. Boîte (500) comprenant une pluralité de couteaux de soudage (4, 4', 56) selon l'une quelconque des revendications 1 à 9 stockés à l'intérieur.

11. Boîte (500) selon la revendication 10, **caractérisée en ce que** les couteaux de soudage individuels (4, 4', 56) sont rangés dans la boîte (500) dans la direction longitudinale.

12. Boîte (500) revendication 10 ou 11, **caractérisée en ce que** la boîte est ouverte d'un côté et **en ce qu'**un élément de ressort agencé dans la boîte pousse le couteau de soudage prévu pour la prochaine opération de soudage en direction de l'ouverture unilatérale.

13. Boîte (500) selon l'une quelconque des revendications 10 à 12, **caractérisée en ce que** la boîte (500) est interchangeable dans son ensemble.

14. Couteau de soudage (4, 4', 56) selon l'une quelconque des revendications 1 à 9 précédentes, **caractérisé en ce que** le couteau de soudage (4, 4', 56) peut être serré dans deux parties de serrage (43), (44) opposées d'un support de couteau de soudage (40).

15. Couteau de soudage (4, 4', 56) selon la revendication 14, **caractérisé en ce que** le couteau de soudage (4) peut être mis en contact électrique.

16. Couteau de soudage (4, 4', 56) selon la revendication 14 ou 15, **caractérisé en ce que** le couteau de soudage (4, 4', 56) peut être relié à l'une des deux parties de serrage (43, 44) du support de couteau de soudage (40) avec un enroulement secondaire formé d'un seul conducteur d'une unité de transformateur (90) et le couteau de soudage (4) ferme le circuit secondaire de l'unité de transformateur (90).

17. Couteau de soudage (4, 4', 56), selon l'une quelconque des revendications 14 à 16, **caractérisé en ce que** le couteau de soudage (4, 4', 56) peut être mis en contact avec au moins l'une des deux parties de serrage (43, 44) par des pièces de pression montées sur ressort.
